# EUROPEAN PATENT SPECIFICATION

(11) **EP 3 287 170 B1**
(45) Date of publication and mention of the grant of the patent: **12.12.2018**
(21) Application number: 17190377.6
(22) Date of filing: 06.10.2005
(51) Int. Cl.: A61N 7/02, A61B 8/08

(54) **SYSTEM FOR CONTROLLED THERMAL TREATMENT OF HUMAN SUPERFICIAL TISSUE**
SYSTEM ZUR GESTEUERTEN WÄRMEBEHANDLUNG VON MENSCHLICHEM OBERFLÄCHENGEWEBE
SYSTÈME DE TRAITEMENT THERMIQUE CONTRÔLÉ DE TISSUS SUPERFICIELS HUMAINS

(30) Priority: 06.10.2004 US 616754 P
(43) Date of publication of application: 28.02.2018
(62) Divisional of application: 11172027.2
(73) Proprietor: Guided Therapy Systems, L.L.C., Mesa, Arizona 85202-1150 (US)
(72) Inventor: SLAYTON, Michael, H., Tempe, AZ 85283 (US); BARTHE, Peter G., Phoenix, AZ 85048 (US); MAKIN, Inder, Raj, S., Mesa, AZ 85215 (US)
(74) Representative: Wallinger Ricker Schlotter Tostmann

(56) References cited:
- WO-A2-03/096883
- US-A- 5 520 188
- US-A- 5 769 790
- US-A1- 2004 015 106
- US-B1- 6 623 430
- DOUGLAS R DAUM ET AL: "Design and Evaluation of a Feedback Based Phased Array System for Ultrasound Surgery", IEEE TRANSACTIONS ON ULTRASONICS, FERROELECTRICS AND FREQUENCY CONTROL, IEEE, US, vol. 45, no. 2, 1 March 1998 (1998-03-01), pages 431-438, XP011062959, ISSN: 0885-3010, DOI: 10.1109/58.660153

## Description

### Field of Invention

This invention generally relates to therapeutic treatment systems, and more particularly, to a system for controlled thermal injury of human superficial tissue.

### Background of the Invention

Current techniques of therapeutic treatment of human superficial tissue for cosmetic applications utilize several different energy sources. Some exemplary conventional energy sources include ablative and non-ablative lasers, radio frequency (RF) energy, and more recently some ultrasound-based techniques. Current examples of ultrasound-based treatment techniques include those disclosed in Klopotek (U.S. Patent No. 6,113,559 and its related continuation, U.S. Patent No. 6,325,769), Hissong et al. (U.S. Patent No. 6,595,934), and Coleman (U.S. Patent No. 6,692,450).

Klopotek initially suggests in U.S. Patent No. 6,113,559 a method of reducing skin wrinkles by applying a focused ultrasound beam so that the dermis layer is "gently stimulated or irritated" without adversely damaging the outer epidermis by using "dosages that are significantly lower than conventional hyperthermia therapies." The disclosed methodology merely alleges a non-thermal injury since energy is applied for a time duration of only 10 ns - 200 µs at a focal intensity of 500 - 1500 W/cm², i.e. 5 µJ/cm² - 0.3 joule/cm². Despite such a low amount of energy, Klopotek alleges that the tissue temperature would rise to temperatures between 47 - 75 °C, sufficient enough to cause injury as opposed to gently stimulating or irritating. Klopotek later discloses in U.S. Patent No. 6,325,769 the use of pulsed (as opposed to continuous wave) ultrasound, but at the same low focal intensities (500 - 1500 W/cm2) and pulse duration (10 ns - 200 µs), and alleges that such an acoustic excitation will create an acoustic shock wave and cavitation effects in the dermis layer. In reality, it would be extremely difficult if not impossible as collectively taught in the '559 and '769 patents for one skilled in the art to induce such cavitation, temperature rise or shock wave in tissue with such "gentle stimulation or irritation" due to fundamental limits of the thermal capacity of tissue, e.g., the specific heat capacity of skin is approximately 3430 J/kg/K, as well as acoustic wave propagation effects.

Hissong discloses a method of skin rejuvenation at frequencies from 0.5 - 12 MHz in which the step of ablating includes forming a focal lesion "to begin at a beginning margin located 50 - 100 µm below the external surface of the skin" and to have a lesion "depth of 50 - 150 µm," i.e. lesions extending from a depth of 50 µm to 250 µm deep from the skin's surface. Hissong also alleges that heating the skin for a "duration of 2 to 60 seconds" will form the focal lesions. However, a number of shortcomings limit the utility of the Hissong technique.

For example, such a long duration of energy delivery would result in significant thermal diffusion and lesion growth, both laterally and axially, drastically hindering placement of focal lesions over a shallow 50 µm to 250 µm depth. Second, if the highest frequency of Hissong, namely 12 MHz, were considered in an application, then the corresponding wavelength in tissue would be approximately 128 µm. Considering that the depth-of-focus for an acoustic beam profile, i.e., the axial focal beam length, comprises several wavelengths, it is not practical to produce such short/sub-wavelength, thermally induced lesions, such as from 50 to 150 µm in length, for even the tightest, diffraction-limited focusing. Furthermore, at lower frequencies it would be more difficult to produce such short/sub-wavelength, thermally induced lesions. Still further, the use of strong focusing requires relatively large aperture transducers such that the multi-element applicator taught by Hissong would be very large and difficult to acoustically couple over facial skin and neck tissue, and it would be extremely difficult to fuse lesions together as alleged. Finally, lesions restricted to such shallow depths and long treatment times as disclosed by Hissong have a limited scope of utility and clinical throughput, which would be further encumbered by the requirement of maintaining of a hand-held probe stationary to micron levels over a long period of time.

Coleman alleges that focused ultrasound ablation initiated from separate, single elements combined mechanically together at the active surface and forming a multi-element unit with "a plurality of individual ultrasound emitting elements arranged in an array" and alleged "to emit ultrasound energy and focus the emitted ultrasound energy a predetermined distance from ultrasound emitting member." Coleman further teaches "a plurality of individual ultrasound emitting elements enclosed in an array," and configured with "each focusing zone being separate and distinct from one another and located the same fixed predetermined distance outwardly." Finally, Coleman describes forming lesions within the tissue by the "ultrasound emitting elements in said array being selectively, independently actuatable to emit ultrasound energy therefrom and being selectively, independently non-actuatable to not emit ultrasound energy therefrom."

Thus, it appears that Coleman, recognizing a real need for flexibility in lesion forming proposes, is attempting to create various shapes of the lesion by combining separate lesions from fixed-focus single elements housed together in a multi-element transducer array actuated separately. Unfortunately, such a technique is severely limited spatially and temporally as well as in its precision due to a heavy reliance on thermal expansion. Moreover, since the multiple-element array is configured to cover a large area, and the targeted tissue is most often curved, it would be difficult to acoustically couple the focused ultrasound ablation device taught by Coleman. Furthermore, since the focused dish transducer elements or at least flat disks need to be large for good intensity gain, it is necessary to have such elements spaced on the order of a wavelength to achieve good focusing, i.e. high intensity gain, low side lobes and grating lobes, thus making the array cumbersome for operation. Finally, although Coleman attempts to form a planar lesion, the lesion uncontrollably grows vertically as well since such a lesion is formed through the lateral thermal diffusion of the energy. US 5,769,790 discloses a system employing a therapy transducer for producing both high-intensity fields as well as low-intensity fields that transmit energy for imaging, which are received by a separate imaging transducer for producing ultrasound images.

US Patent No. 6,623,430 discloses a method and apparatus for controlling the safe delivery of thermosensitive liposomes containing medicant to a targeted tissue region using ultrasound. Thermosensitive liposomes containing medicants are delivered to a region of interest, the region of interest is located using ultrasound imaging, ultrasound therapy is applied to heat the region of interest, and the temperature of the region is monitored to determine whether a designated threshold temperature has been reached which allows for the release of medicants from the liposomes. If the threshold temperature is reached, and the liposomes are melted, the treatment stops. If the threshold temperature has not been reached, the application of ultrasound therapy and ultrasound imaging are alternated until the threshold temperature is reached. The ultrasound imaging, temperature monitoring and ultrasound therapy are preferably performed with a single transducer.

US Patent No. 5,520,188 discloses a transducer for use in a localization and therapeutic ultrasound system. The transducer of the present invention includes multiple elements that are driven separately. The elements operate together to focus a continuous wave ultrasound beam at a focal zone that is at a variable distance from the elements. The transducer includes a mechanism to adjust the focal distance so that the focal zone may be moved to multiple depths.

DAUM R ET AL: "Design and evaluation of a feedback based phased array system for ultrasound surgery" IEEE TRANSACTIONS ON ULTRASONICS, FERROELECTRICS AND FREQUENCY CONTROL IEEE USA, vol. 45, no. 2, March 1998 (1998-03), pages 431-438, XP002362996 ISSN: 0885-3010 discloses that a feedback loop may be used with phased array ultrasound applicators. This feedback loop appears to reduce the RF electrical power variation from into a 16 element array. This feedback loop has been experimentally shown to increase the focal intensities by of two tested phased arrays without array calibration using a hydrophone.

Accordingly, conventional therapeutic treatment techniques have numerous fundamental physical limits, technological difficulties, and practical utility issues that prevent the flexible, precise creation and control of lesions of arbitrary shape, size and depth within human superficial tissue.

### Summary of the Invention

The present invention is directed towards a therapeutic treatment system, aspects of which are set out in the appended claim set.

### Brief Description of the Drawings

The subject matter of the invention is particularly pointed out and distinctly claimed in the concluding portion of the specification. The invention, however, both as to structure and method of operation, may best be understood by reference to the following description taken in conjunction with the claims and the accompanying drawing figures, in which like parts may be referred to by like numerals:
FIG. 1 illustrates a block diagram of an exemplary therapeutic treatment system for controlled thermal injury of human superficial tissue in accordance with an exemplary embodiment of the present invention;
FIG. 2 illustrates a cross sectional diagram of a human superficial tissue region of interest including a plurality of lesions of controlled thermal injury in accordance with an exemplary embodiment of the present invention;
FIGS. 3A and 3B illustrate block diagrams of an exemplary control system in accordance with exemplary embodiments of the present invention;
FIGS. 4A and 4B illustrate block diagrams of an exemplary probe system in accordance with exemplary embodiments of the present invention;
FIG. 5 illustrates a cross-sectional diagram of an exemplary transducer, not forming part of the present invention;
FIGS. 6A and 6B illustrate cross-sectional diagrams of an exemplary transducer, not forming part of the present invention;
FIG. 7 illustrates exemplary transducer configurations for ultrasound treatment, not all forming part of the present invention;
FIGS. 8A and 8B illustrate cross-sectional diagrams of an exemplary transducer in accordance with another exemplary embodiment of the present invention;
FIG. 9 illustrates an exemplary transducer configured as a two-dimensional array for ultrasound treatment, not forming part of the present invention;
FIGS. 10A-10F illustrate cross-sectional diagrams of exemplary transducers, not forming part of the present invention;
FIG. 11 illustrates a schematic diagram of an acoustic coupling and cooling system in accordance with an exemplary embodiment of the present invention;
FIGS. 12A and 12B illustrate block diagrams of exemplary open-loop and closed-loop feedback systems in accordance exemplary embodiments of the present invention;
FIG. 13 illustrates an exemplary diagram of simulation results for various spatially controlled configurations in accordance with exemplary embodiments of the present invention;
FIG. 14 illustrates an exemplary diagram of simulation results of a pair of lesioning and simulation results in accordance with the present invention; and
FIG. 15 illustrates another exemplary diagram of simulation results of a pair of lesioning results in accordance with the present invention.

### Detailed Description

The present invention may be described herein in terms of various components and processing steps. It should be appreciated that such components and steps may be realized by any number of hardware components configured to perform the specified functions. For example, the present invention may employ various medical treatment devices, visual imaging and display devices, input terminals and the like, which may carry out a variety of functions under the control of one or more control systems or other control devices. In addition, the present invention may be practiced in any number of medical or treatment contexts and the exemplary embodiments relating to a therapeutic treatment method and system for controlled thermal injury of human superficial tissue as described herein are merely a few of the exemplary applications for the invention. For example, the principles, features and methods discussed may be applied to any other medical or other tissue or treatment application.

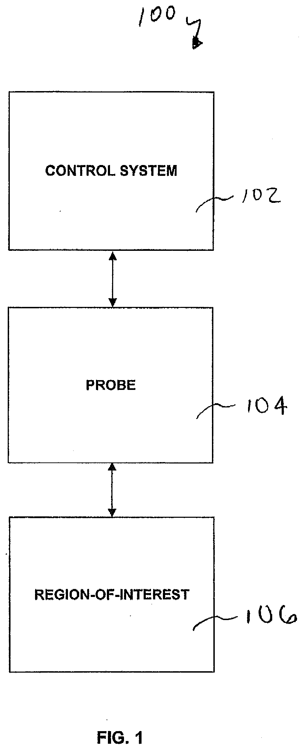

In accordance with various aspects of the present invention, a therapeutic treatment system for controlled thermal injury of human superficial tissue is based on the ability to controllably create thermal lesions of conformally variable shape, size, and depth through precise spatial and temporal control of acoustic energy deposition. With reference to FIG. 1, in accordance with an exemplary embodiment, an exemplary therapeutic treatment system 100 includes a control system 102 and a probe system 104 that can facilitate treatment planning, controlling and/or delivering of acoustic energy, and/or monitoring of treatment conditions to a region of interest 106. Region-of-interest 106 is configured within the human superficial tissue comprising from just below the tissue outer surface to approximately 30 mm or more in depth.

Therapeutic treatment system 100 is configured with the ability to controllably produce conformal lesions of thermal injury in superficial human tissue within region of interest 106 through precise spatial and temporal control of acoustic energy deposition, i.e., control of probe 104 is confined within selected time and space parameters, with such control being independent of the tissue. In accordance with an exemplary embodiment, control system 102 and probe system 104 can be suitably configured for spatial control of the acoustic energy by controlling the manner of distribution of the acoustical energy. For example, spatial control may be realized through selection of the type of one or more transducer configurations insonifying region of interest 106, selection of the placement and location of probe system 104 for delivery of acoustical energy relative to region-of-interest 106, e.g., probe system 104 being configured for scanning over part or whole of region-of-interest 106 to produce contiguous thermal injury having a particular orientation or otherwise change in distance from region-of-interest 106, and/or control of other environment parameters, e.g., the temperature at the acoustic coupling interface can be controlled, and/or the coupling of probe 104 to human tissue. In addition to the spatial control parameters, control system 102 and probe system 104 can also be configured for temporal control, such as through adjustment and optimization of drive amplitude levels, frequency/waveform selections, e.g., the types of pulses, bursts or continuous waveforms, and timing sequences and other energy drive characteristics to control thermal ablation of tissue. The spatial and/or temporal control can also be facilitated through open-loop and closed-loop feedback arrangements, such as through the monitoring of various spatial and temporal characteristics. As a result, control of acoustical energy within six degrees of freedom, e.g., spatially within the X, Y and Z domain, as well as the axis of rotation within the XY, YZ and XZ domains, can be suitably achieved to generate conformal lesions of variable shape, size and orientation.

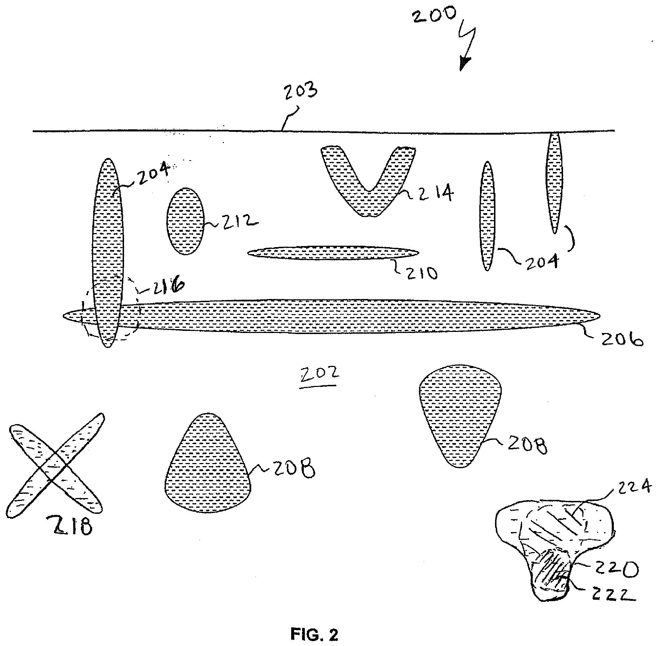

For example, through such spatial and/or temporal control, an exemplary treatment system 100 can enable the regions of thermal injury to possess arbitrary shape and size and allow the tissue to be destroyed (ablated) in a controlled manner. With reference to FIG. 2, one or more thermal lesions may be created within a tissue region of interest 200, with such thermal lesions having a narrow or wide lateral extent, long or short axial length, and/or deep or shallow placement, including up to a tissue outer surface 203. For example, cigar shaped lesions may be produced in a vertical disposition 204 and/or horizontal disposition 206. In addition, raindrop-shaped lesions 208, flat planar lesions 210, round lesions 212 and/or other v-shaped/ellipsoidal lesions 214 may be formed, among others. For example, mushroom-shaped lesion 220 may be provided, such as through initial generation of a an initial round or cigar-shaped lesion 222, with continued application of ablative ultrasound resulting in thermal expansion to further generate a growing lesion 224, such thermal expansion being continued until mushroom-shaped lesion 220 is achieved. The plurality of shapes can also be configured in various sizes and orientations, e.g., lesions 208 could be rotationally oriented clockwise or counterclockwise at any desired angle, or made larger or smaller as selected, all depending on spatial and/or temporal control. Moreover, separate islands of destruction, i.e., multiple lesions separated throughout the tissue region, may also be created over part of or the whole portion within tissue region-of-interest 200. In addition, contiguous structures and/or overlapping structures 216 may be provided from the controlled configuration of discrete lesions. For example, a series of one or more crossed-lesions 218 can be generated along a tissue region to facilitate various types of treatment methods.

The specific configurations of controlled thermal injury are selected to achieve the desired tissue and therapeutic effect(s). For example, any tissue effect can be realized, including but not limited to thermal and non-thermal streaming, cavitational, hydrodynamic, ablative, hemostatic, diathermic, and/or resonance-induced tissue effects. Such effects can be suitably realized at treatment depths over a range of approximately 0-30000 µm within region of interest 200 to provide a high degree of utility.

With reference again to FIG. 1, an exemplary therapeutic treatment system 100 comprising control system 102 and probe system 104 may also comprise various configurations and can be subdivided into various separate subsystems and components. For example, therapeutic treatment system 100 may be divided into various system and probe components disposed in a suitable position for facilitating spatial and/or temporal distribution of acoustical energy. In addition, control system 102 and probe system 104 can comprise other subsystems, such as an imaging subsystem within control system 102 configured to operate and control an imaging transducer within probe system 104, e.g., a separate imaging transducer and a separate therapy transducer or a combined imaging/therapy transducer configured for tissue parameter monitoring.

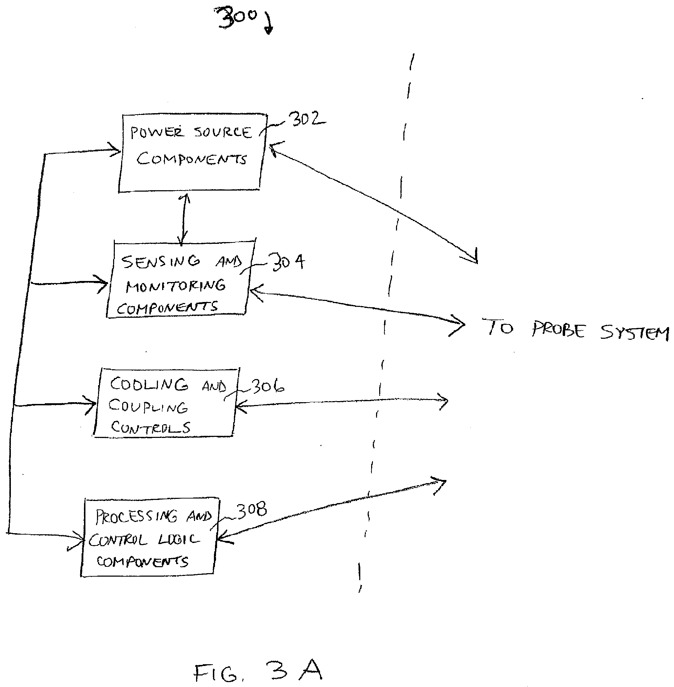

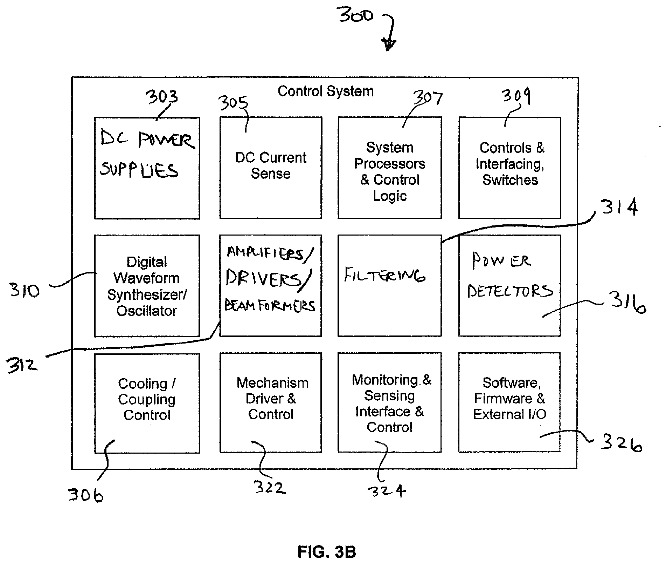

With reference to FIGS. 3A and 3B, in accordance with exemplary embodiments, an exemplary control system 300 can be configured for coordination and control of the entire therapeutic treatment process in accordance with the adjustable settings made by a therapeutic treatment system user. For example, control system 300 can suitably comprise power source components 302, sensing and monitoring components 304, cooling and coupling controls 306, and/or processing and control logic components 308. Control system 300 can be configured and optimized in a variety of ways with more or less subsystems and components to implement the therapeutic system for controlled thermal injury, and the embodiment in FIGS. 3A and 3B are merely for illustration purposes.

For example, for power sourcing components 302, control system 300 can comprise one or more direct current (DC) power supplies 303 configured to provide electrical energy for entire control system 300, including power required by a transducer electronic amplifier/driver 312. A DC current sense device 305 can also be provided to confirm the level of power going into amplifiers/drivers 312 for safety and monitoring purposes.

Amplifiers/drivers 312 can comprise multi-channel or single channel power amplifiers and/or drivers. In accordance with an exemplary embodiment for transducer array configurations, amplifiers/drivers 312 can also be configured with a beamformer to facilitate array focusing. An exemplary beamformer can be electrically excited by an oscillator/digitally controlled waveform synthesizer 310 with related switching logic.

The power sourcing components can also include various filtering configurations 314. For example, switchable harmonic filters and/or matching may be used at the output of amplifier/driver 312 to increase the drive efficiency and effectiveness. Power detection components 316 may also be included to confirm appropriate operation and calibration. For example, electric power and other energy detection components 316 may be used to monitor the amount of power going to an exemplary probe system.

Various sensing and monitoring components 304 may also be suitably implemented within control system 300. For example, in accordance with an exemplary embodiment, monitoring, sensing and interface control components 324 may be configured to operate with various motion detection systems implemented within transducer probe 104 to receive and process information such as acoustic or other spatial and temporal information from a region of interest. Sensing and monitoring components can also include various controls, interfacing and switches 309 and/or power detectors 316. Such sensing and monitoring components 304 can facilitate open-loop and/or closed-loop feedback systems within treatment system 100. Still further, monitoring, sensing and interface control components 324 may comprise imaging systems configured for one-dimensional, two-dimensional and/or three dimensional imaging functions. Such imaging systems can comprise any imaging modality based on at least one of photography and other visual optical methods, magnetic resonance imaging (MRI), computed tomography (CT), optical coherence tomography (OCT), electromagnetic, microwave, or radio frequency (RF) methods, positron emission tomography (PET), infrared, ultrasound, acoustic, or any other suitable method of visualization, localization, or monitoring of a region-of-interest 106. Still further, various other tissue parameter monitoring components, such as temperature measuring devices and components, can be configured within monitoring, sensing and interface control components 324, such monitoring devices comprising any modality now known or hereinafter devised.

Cooling/coupling control systems 306 may be provided to remove waste heat from an exemplary probe 104, provide a controlled temperature at the superficial tissue interface and deeper into the tissue, and/or provide acoustic coupling from transducer probe 104 to region-of-interest 106. Such cooling/coupling control systems 306 can also be configured to operate in both open-loop and/or closed-loop feedback arrangements with various coupling and feedback components.

Processing and control logic components 308 can comprise various system processors and digital control logic 307, such as one or more of microcontrollers, microprocessors, field-programmable gate arrays (FPGAs), computer boards, and associated components, including firmware and control software 326, which interfaces to user controls and interfacing circuits as well as input/output circuits and systems for communications, displays, interfacing, storage, documentation, and other useful functions. System software and firmware 326 controls all initialization, timing, level setting, monitoring, safety monitoring, and all other system functions required to accomplish user-defined treatment objectives. Further, various control switches 308 can also be suitably configured to control operation.

An exemplary transducer probe 104 can also be configured in various manners and comprise a number of reusable and/or disposable components and parts in various embodiments to facilitate its operation. For example, transducer probe 104 can be configured within any type of transducer probe housing or arrangement for facilitating the coupling of transducer to a tissue interface, with such housing comprising various shapes, contours and configurations depending on the particular treatment application. For example, in accordance with an exemplary embodiment, transducer probe 104 can be depressed against a tissue interface whereby blood perfusion is partially or completely cut-off, and tissue flattened in superficial treatment region-of-interest 106. Transducer probe 104 can comprise any type of matching, such as for example, electric matching, which may be electrically switchable; multiplexer circuits and/or aperture/element selection circuits; and/or probe identification devices, to certify probe handle, electric matching, transducer usage history and calibration, such as one or more serial EEPROM (memories). Transducer probe 104 may also comprise cables and connectors; motion mechanisms, motion sensors and encoders; thermal monitoring sensors; and/or user control and status related switches, and indicators such as LEDs. For example, a motion mechanism in probe 104 may be used to controllably create multiple lesions, or sensing of probe motion itself may be used to controllably create multiple lesions and/or stop creation of lesions, e.g. for safety reasons if probe 104 is suddenly jerked or is dropped. In addition, an external motion encoder arm may be used to hold the probe during use, whereby the spatial position and attitude of probe 104 is sent to the control system to help controllably create lesions. Furthermore, other sensing functionality such as profilometers or other imaging modalities may be integrated into the probe in accordance with various exemplary embodiments.

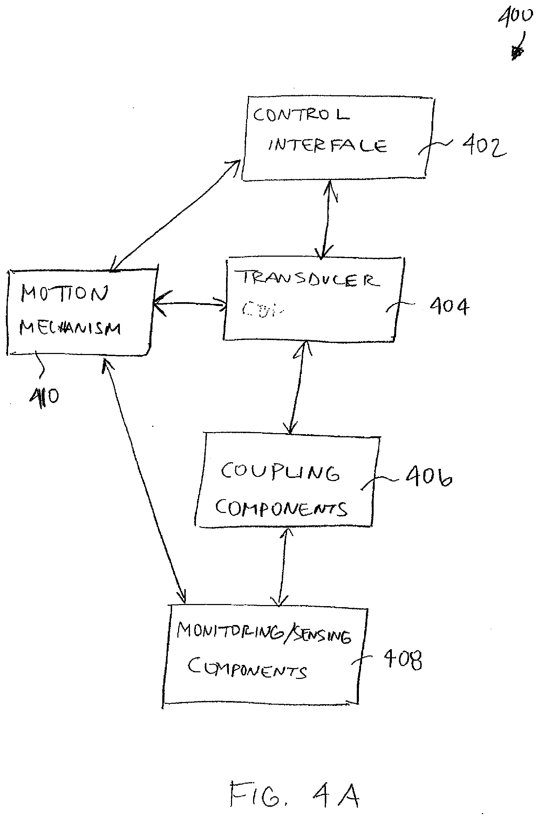

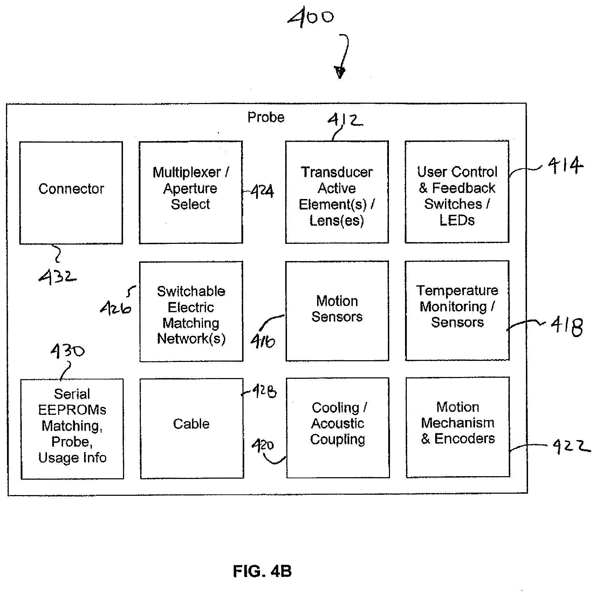

With reference to FIGS. 4A and 4B, in accordance with an exemplary embodiment, a transducer probe 400 can comprise a control interface 402, a transducer 404, coupling components 406, and monitoring/sensing components 408, and/or motion mechanism 410. However, transducer probe 400 can be configured and optimized in a variety of ways with more or less parts and components to provide ultrasound energy for controlled thermal injury, and the embodiment in FIGS. 4A and 4B are merely for illustration purposes.

Control interface 402 is configured for interfacing with control system 300 to facilitate control of transducer probe 400. Control interface components 402 can comprise multiplexer/aperture select 424, switchable electric matching networks 426, serial EEPROMs and/or other processing components and matching and probe usage information 430 and interface connectors 432.

Coupling components 406 can comprise various devices to facilitate coupling of transducer probe 400 to a region of interest. For example, coupling components 406 can comprise cooling and acoustic coupling system 420 configured for acoustic coupling of ultrasound energy and signals. Acoustic cooling/coupling system 420 with possible connections such as manifolds may be utilized to couple sound into the region-of-interest, control temperature at the interface and deeper into tissue, provide liquid-filled lens focusing, and/or to remove transducer waste heat. Coupling system 420 may facilitate such coupling through use of various coupling mediums, including air and other gases, water and other fluids, gels, solids, and/or any combination thereof, or any other medium that allows for signals to be transmitted between transducer active elements 412 and a region of interest. In addition to providing a coupling function, in accordance with an exemplary embodiment, coupling system 420 can also be configured for providing temperature control during the treatment application. For example, coupling system 420 can be configured for controlled cooling of an interface surface or region between transducer probe 400 and a region of interest and deeper into tissue by suitably controlling the temperature of the coupling medium. The suitable temperature for such coupling medium can be achieved in various manners, and utilize various feedback systems, such as thermocouples, thermistors or any other device or system configured for temperature measurement of a coupling medium. Such controlled cooling can be configured to further facilitate spatial and/or thermal energy control of transducer probe 400.

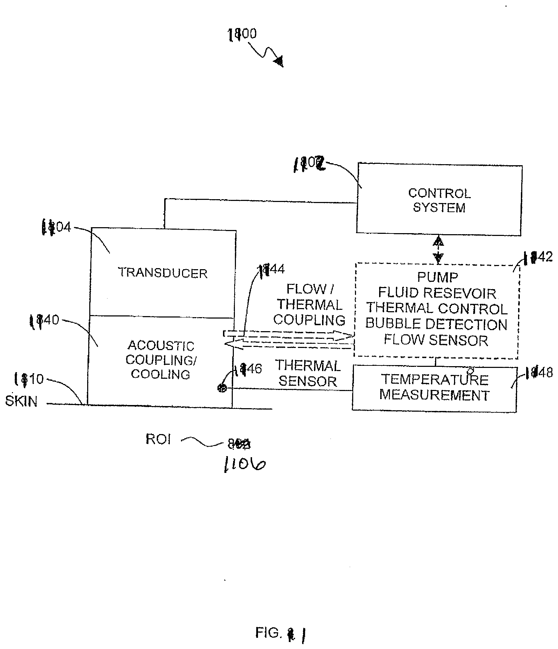

In accordance with an exemplary embodiment, with additional reference to Fig. 11, acoustic coupling and cooling 1140 can be provided to acoustically couple energy and imaging signals from transducer probe 1104 to and from the region of interest 1106, to provide thermal control at the probe to region-of-interest interface 1110 and deeper into the tissue to control lesioning, and to remove potential waste heat from the transducer probe at region 1144. Temperature monitoring can be provided at the coupling interface via a thermal sensor 1146 to provides a mechanism of temperature measurement 1148 and control via control system 1102 and a thermal control system 1142. Thermal control may consist of passive cooling such as via heat sinks or natural conduction and convection or via active cooling such as with peltier thermoelectric coolers, refrigerants, or fluid-based systems comprised of pump, fluid reservoir, bubble detection, flow sensor, flow channels/tubing 1144 and thermal control 1142.

With continued reference to Fig. 4, monitoring and sensing components 408 can comprise various motion and/or position sensors 416, temperature monitoring sensors 418, user control and feedback switches 414 and other like components for facilitating control by control system 300, e.g., to facilitate spatial and/or temporal control through open-loop and closed-loop feedback arrangements that monitor various spatial and temporal characteristics.

Motion mechanism 410 can comprise manual operation, mechanical arrangements, or some combination thereof. For example, a motion mechanism 422 can be suitably controlled by control system 300, such as through the use of accelerometers, encoders or other position/orientation devices 416 to determine and enable movement and positions of transducer probe 400. Linear, rotational or variable movement can be facilitated, e.g., those depending on the treatment application and tissue contour surface.

Transducer 404 can comprise one or more transducers configured for producing conformal lesions of thermal injury in superficial human tissue within a region of interest through precise spatial and temporal control of acoustic energy deposition. Transducer 404 can also comprise one or more transduction elements and/or lenses 412. The transduction elements can comprise a piezoelectrically active material, such as lead zirconante titanate (PZT), or any other piezoelectrically active material, such as a piezoelectric ceramic, crystal, plastic, and/or composite materials, as well as lithium niobate, lead titanate, barium titanate, and/or lead metaniobate. In addition to, or instead of, a piezoelectrically active material, transducer 404 can comprise any other materials configured for generating radiation and/or acoustical energy. Transducer 404 can also comprise one or more matching layers configured along with the transduction element such as coupled to the piezoelectrically active material. Acoustic matching layers and/or damping may be employed as necessary to achieve the desired electroacoustic response.

In accordance with an exemplary embodiment, the thickness of the transduction element of transducer 404 can be configured to be uniform. That is, a transduction element 412 can be configured to have a thickness that is substantially the same throughout. In accordance with another exemplary embodiment, the thickness of a transduction element 412 can also be configured to be variable. For example, transduction element(s) 412 of transducer 404 can be configured to have a first thickness selected to provide a center operating frequency of a lower range, for example from approximately 1 kHz to 3 MHz. Transduction element 404 can also be configured with a second thickness selected to provide a center operating frequency of a higher range, for example from approximately 3 to 100 MHz or more. Transducer 404 can be configured as a single broadband transducer excited with at least two or more frequencies to provide an adequate output for generating a desired response. Transducer 404 can also be configured as two or more individual transducers, wherein each transducer comprises one or more transduction element. The thickness of the transduction elements can be configured to provide center-operating frequencies in a desired treatment range. For example, transducer 404 can comprise a first transducer configured with a first transduction element having a thickness corresponding to a center frequency range of approximately 1 kHz to 3 MHz, and a second transducer configured with a second transduction element having a thickness corresponding to a center frequency of approximately 3 MHz to 100 MHz or more.

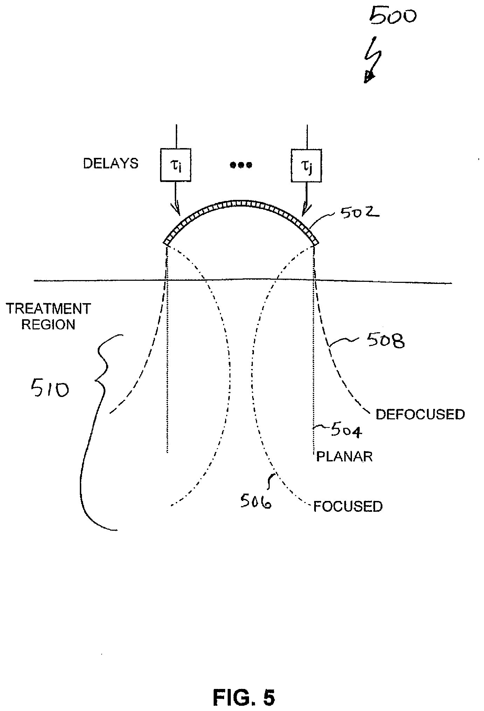

Transducer 404 may be composed of one or more individual transducers in any combination of focused, planar, or unfocused single-element, multi-element, or array transducers, including 1-D, 2-D, and annular arrays; linear, curvilinear, sector, or spherical arrays; spherically, cylindrically, and/or electronically focused, defocused, and/or lensed sources. For example, with reference to an exemplary embodiment depicted in Fig. 5, transducer 500 can be configured as an acoustic array to facilitate phase focusing. That is, transducer 500 can be configured as an array of electronic apertures that may be operated by a variety of phases via variable electronic time delays. By the term "operated," the electronic apertures of transducer 500 may be manipulated, driven, used, and/or configured to produce and/or deliver an energy beam corresponding to the phase variation caused by the electronic time delay. For example, these phase variations can be used to deliver defocused beams, planar beams, and/or focused beams, each of which may be used in combination to achieve different physiological effects in a region of interest 510. Transducer 500 may additionally comprise any software and/or other hardware for generating, producing and or driving a phased aperture array with one or more electronic time delays.

Transducer 500 can also be configured to provide focused treatment to one or more regions of interest using various frequencies. In order to provide focused treatment, transducer 500 can be configured with one or more variable depth devices to facilitate treatment. For example, transducer 500 may be configured with variable depth devices disclosed in U.S. Patent Application 10/944,500, published as US 2006/0058664 A1. In addition, transducer 500 can also be configured to treat one or more additional ROI 510 through the enabling of sub-harmonics or pulse-echo imaging, as disclosed in U.S. Patent Application 10/944,499, published as US 2006/0058717 A1.

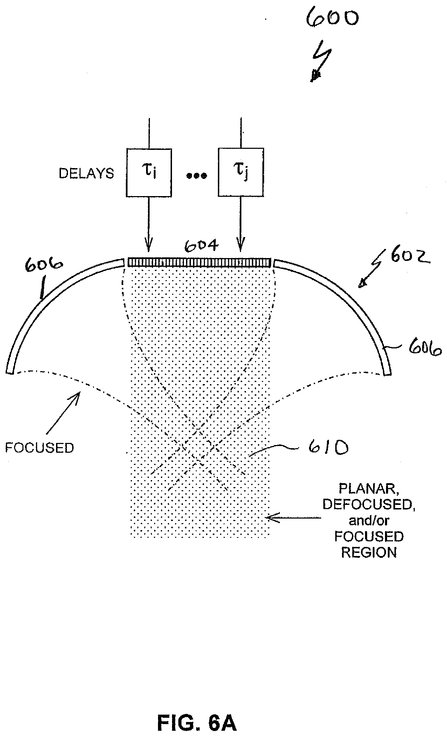

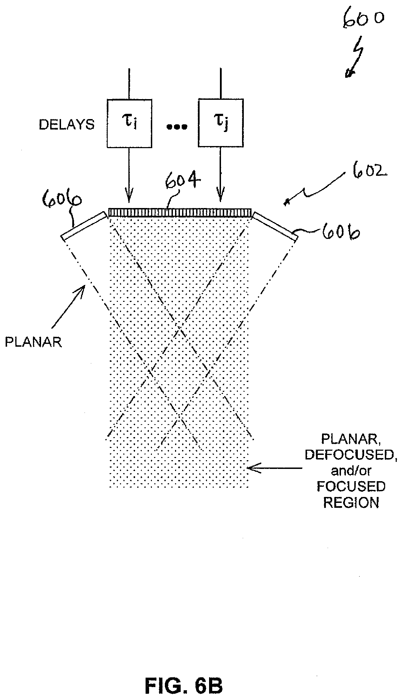

Moreover, any variety of mechanical lenses or variable focus lenses, e.g. liquid-filled lenses, may also be used to focus and or defocus the sound field. For example, with reference to exemplary embodiments depicted in Figs. 6A and 6B, transducer 600 may also be configured with an electronic focusing array 604 in combination with one or more transduction elements 606 to facilitate increased flexibility in treating ROI 610. Array 604 may be configured in a manner similar to transducer 502. That is, array 604 can be configured as an array of electronic apertures that may be operated by a variety of phases via variable electronic time delays, for example, T₁, T₂...Tⱼ. By the term "operated," the electronic apertures of array 604 may be manipulated, driven, used, and/or configured to produce and/or deliver energy in a manner corresponding to the phase variation caused by the electronic time delay. For example, these phase variations can be used to deliver defocused beams, planar beams, and/or focused beams, each of which may be used in combination to achieve different physiological effects in ROI 610.

Transduction elements 606 may be configured to be concave, convex, and/or planar. For example, in an exemplary embodiment depicted in Fig. 6A, transduction elements 606A are configured to be concave in order to provide focused energy for treatment of ROI 610. Additional embodiments are disclosed in U.S. Patent Application 10/944,500, published as US 2006/0058664 A1.

In another exemplary embodiment, depicted in Fig. 6B, transduction elements 606B can be configured to be substantially flat in order to provide substantially uniform energy to ROI 610. While Figs. 6A and 6B depict exemplary embodiments with transduction elements 604 configured as concave and substantially flat, respectively, transduction elements 604 can be configured to be concave, convex, and/or substantially flat. In addition, transduction elements 604 can be configured to be any combination of concave, convex, and/or substantially flat structures. For example, a first transduction element can be configured to be concave, while a second transduction element can be configured to be substantially flat.

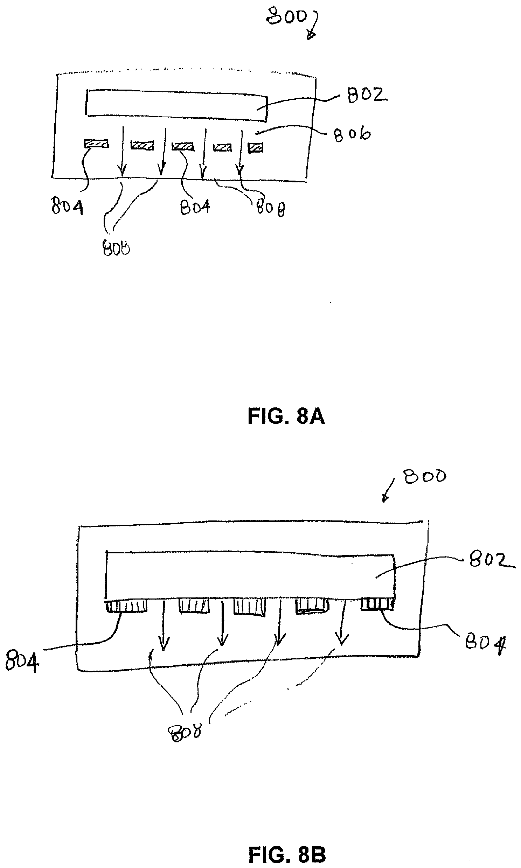

With reference to Figs. 8A and 8B, transducer 404 can be configured as single-element arrays, wherein a single-element 802, e.g., a transduction element of various structures and materials, can be configured with a plurality of masks 804, such masks comprising ceramic, metal or any other material or structure for masking or altering energy distribution from element 802, creating an array of energy distributions 808. Masks 804 can be coupled directly to element 802 or separated by a standoff 806, such as any suitably solid or liquid material.

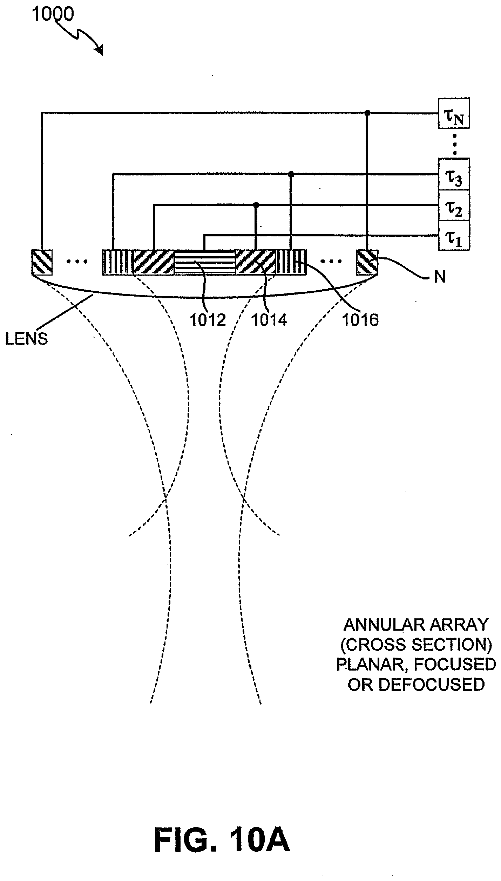

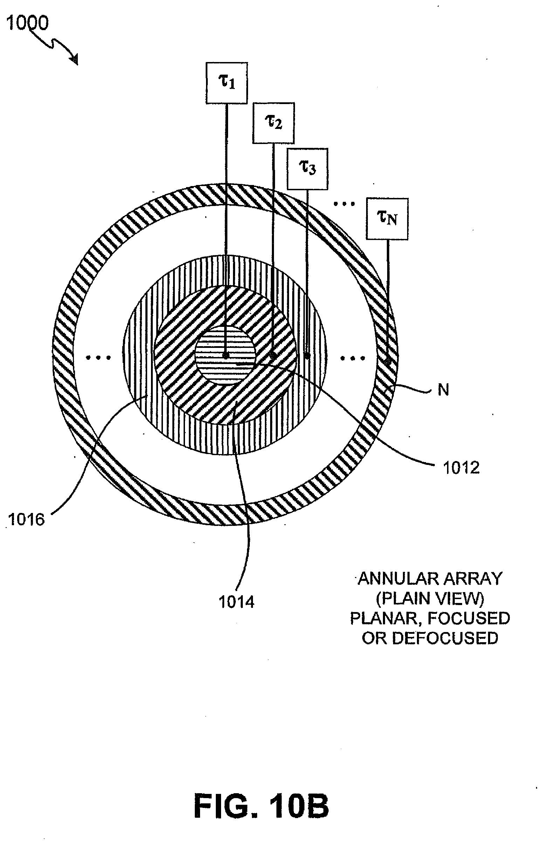

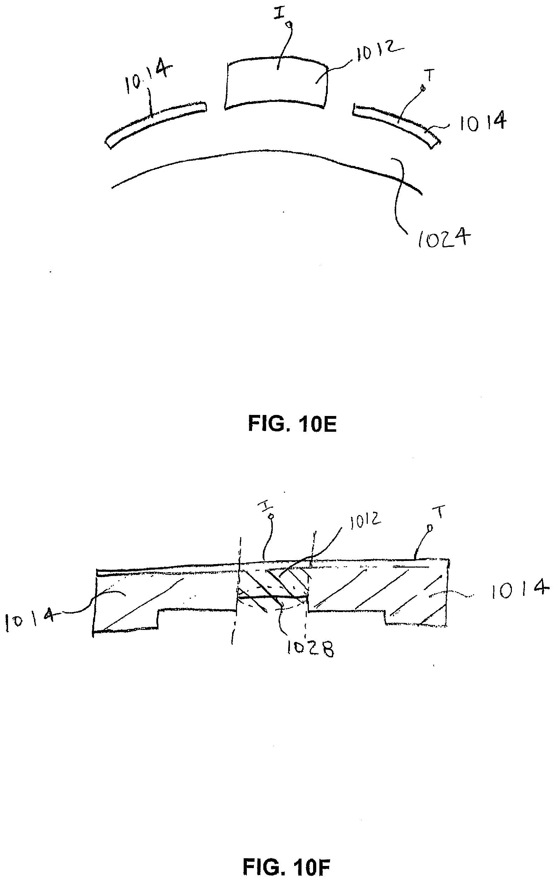

An exemplary transducer 404 can also be configured as an annular array to provide planar, focused and/or defocused acoustical energy. For example, with reference to Figs. 10A and 10B, in accordance with an exemplary embodiment, an annular array 1000 can comprise a plurality of rings 1012, 1014, 1016 to N. Rings 1012, 1014, 1016 to N can be mechanically and electrically isolated into a set of individual elements, and can create planar, focused, or defocused waves. For example, such waves can be centered on-axis, such as by methods of adjusting corresponding transmit and/or receive delays, τ₁, τ₂, τ₃ ... τ_{N}. An electronic focus can be suitably moved along various depth positions, and can enable variable strength or beam tightness, while an electronic defocus can have varying amounts of defocusing. In accordance with an exemplary embodiment, a lens and/or convex or concave shaped annular array 1000 can also be provided to aid focusing or defocusing such that any time differential delays can be reduced. Movement of annular array 800 in one, two or three-dimensions, or along any path, such as through use of probes and/or any conventional robotic arm mechanisms, may be implemented to scan and/or treat a volume or any corresponding space within a region of interest.

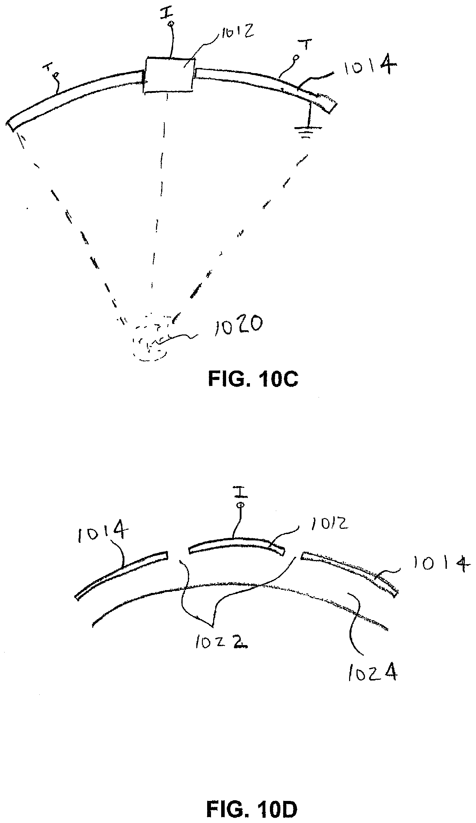

Transducer 404 can also be configured in other annular or non-array configurations for imaging/therapy functions. For example, with reference to Figs. 10C-10F, a transducer can comprise an imaging element 1012 configured with therapy element(s) 1014. Elements 1012 and 1014 can comprise a single-transduction element, e.g., a combined imaging/therapy element, or separate elements, such as an imaging element 1012 configured within a hole or opening between therapy elements 1014 as illustrated in Fig. 10C, can be electrically isolated 1022 within the same transduction element or between separate imaging and therapy elements such as illustrated in Fig. 10D, and/or can comprise standoff 1024 or other matching layers, or any combination thereof. For example, with particular reference to Fig. 10F, a transducer can comprise an imaging element 1012 having a surface 1028 configured for focusing, defocusing or planar energy distribution, with therapy elements 1014 including a stepped-configuration lens configured for focusing, defocusing, or planar energy distribution.

In accordance with various exemplary embodiments of the present invention, transducer 404 may be configured to provide one, two and/or three-dimensional treatment applications for focusing acoustic energy to one or more regions of interest. For example, as discussed above, transducer 404 can be suitably diced to form a one-dimensional array, e.g., transducer 602 comprising a single array of sub-transduction elements.

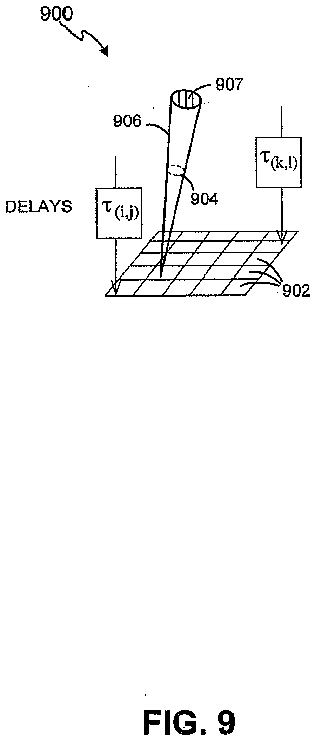

In accordance with another exemplary embodiment, transducer 404 may be suitably diced in two-dimensions to form a two-dimensional array. For example, with reference to Fig. 9, an exemplary two-dimensional array 900 can be suitably diced into a plurality of two-dimensional portions 902. Two-dimensional portions 902 can be suitably configured to focus on the treatment region at a certain depth, and thus provide respective slices 904 of the treatment region. As a result, the two-dimensional array 900 can provide a two-dimensional slicing of the image place of a treatment region, thus providing two-dimensional treatment. In accordance with another exemplary embodiment, transducer 404 may be suitably configured to provide three-dimensional treatment. For example, to provide-three dimensional treatment of a region of interest, with reference again to Fig. 1, a three-dimensional system can comprise a transducer within probe 104 configured with an adaptive algorithm, such as, for example, one utilizing three-dimensional graphic software, contained in a control system, such as control system 102. The adaptive algorithm is suitably configured to receive two-dimensional imaging, temperature and/or treatment or other tissue parameter information relating to the region of interest, process the received information, and then provide corresponding three-dimensional imaging, temperature and/or treatment information.

In accordance with an exemplary embodiment, with reference again to Fig. 9, an exemplary three-dimensional system can comprise a two-dimensional array 900 configured with an adaptive algorithm to suitably receive 904 slices from different image planes of the treatment region, process the received information, and then provide volumetric information 906, e.g., three-dimensional imaging, temperature and/or treatment information. Moreover, after processing the received information with the adaptive algorithm, the two-dimensional array 900 may suitably provide therapeutic heating to the volumetric region 906 as desired.

In accordance with other exemplary embodiments, rather than utilizing an adaptive algorithm, such as three-dimensional software, to provide three-dimensional imaging and/or temperature information, an exemplary three-dimensional system can comprise a single transducer 404 configured within a probe arrangement to operate from various rotational and/or translational positions relative to a target region.

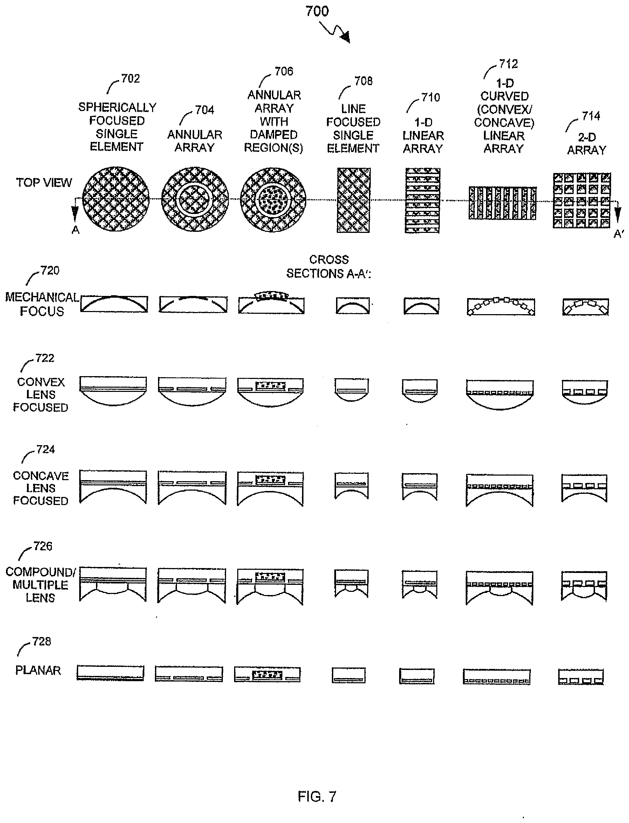

To further illustrate the various structures for transducer 404, with reference to Figure 7, ultrasound therapy transducer 700 can be configured for a single focus, an array of foci, a locus of foci, a line focus, and/or diffraction patterns. Transducer 700 can also comprise single elements, multiple elements, annular arrays, one-, two-, or three-dimensional arrays, broadband transducers, and/or combinations thereof, with or without lenses, acoustic components, and mechanical and/or electronic focusing. Transducers configured as spherically focused single elements 702, annular arrays 704, annular arrays with damped regions 706, line focused single elements 708, 1-D linear arrays 710, 1-D curvilinear arrays in concave or convex form, with or without elevation focusing, 2-D arrays, and 3-D spatial arrangements of transducers may be used to perform therapy and/or imaging and acoustic monitoring functions. For any transducer configuration, focusing and/or defocusing may be in one plane or two planes via mechanical focus 720, convex lens 722, concave lens 724, compound or multiple lenses 726, planar form 728, or stepped form, such as illustrated in Fig. 10F. Any transducer or combination of transducers may be utilized for treatment. For example, an annular transducer may be used with an outer portion dedicated to therapy and the inner disk dedicated to broadband imaging wherein such imaging transducer and therapy transducer have different acoustic lenses and design, such as illustrated in Fig. 10C-10F.

With a better understanding of the various transducer structures, and with reference again to Figure 2, how the geometric configuration of the transducer or transducers that contributes to the wide range of lesioning effects can be better understood. For example, cigar-shaped lesions 204 and 206 may be produced from a spherically focused source, and/or planar lesions 210 from a flat source. Concave planar sources and arrays can produce a "V-shaped" or ellipsoidal lesion 214. Electronic arrays, such as a linear array, can produce defocused, planar, or focused acoustic beams that may be employed to form a wide variety of additional lesion shapes at various depths. An array may be employed alone or in conjunction with one or more planar or focused transducers. Such transducers and arrays in combination produce a very wide range of acoustic fields and their associated benefits. A fixed focus and/or variable focus lens or lenses may be used to further increase treatment flexibility. A convex-shaped lens, with acoustic velocity less than that of superficial tissue, may be utilized, such as a liquid-filled lens, gel-filled or solid gel lens, rubber or composite lens, with adequate power handling capacity; or a concave-shaped, low profile, lens may be utilized and composed of any material or composite with velocity greater than that of tissue. While the structure of transducer source and configuration can facilitate a particular shaped lesion as suggested above, such structures are not limited to those particular shapes as the other spatial parameters, as well as the temporal parameters, can facilitate additional shapes within any transducer structure and source.

The physiological effects created in tissue are not only affected by the spatial distribution of energy, such as transducer structure, distance/placement, orientation, and/or movement, but also its temporal, time-varying characteristics. For example, as to temporal control, each array, two-dimensional array, or single element transducer may be used at various transmit frequencies, and may be either broadband or relatively narrowband, with center frequencies ranging from approximately 1 MHz to 40 MHz, or even with single broadband pulses of energy. Amplitude levels and frequency selection may be changed during treatment to further enhance options. Transmit duration and energy levels are configured to overcome tissue thermal capacity and create controlled thermal injury (necrosis) and/or ablation. Thermal capacity is the minimum amount of energy/heat that is sufficient for live tissue to lose function. In this context, thermal capacity is the minimum amount of energy to result in live tissue destruction.

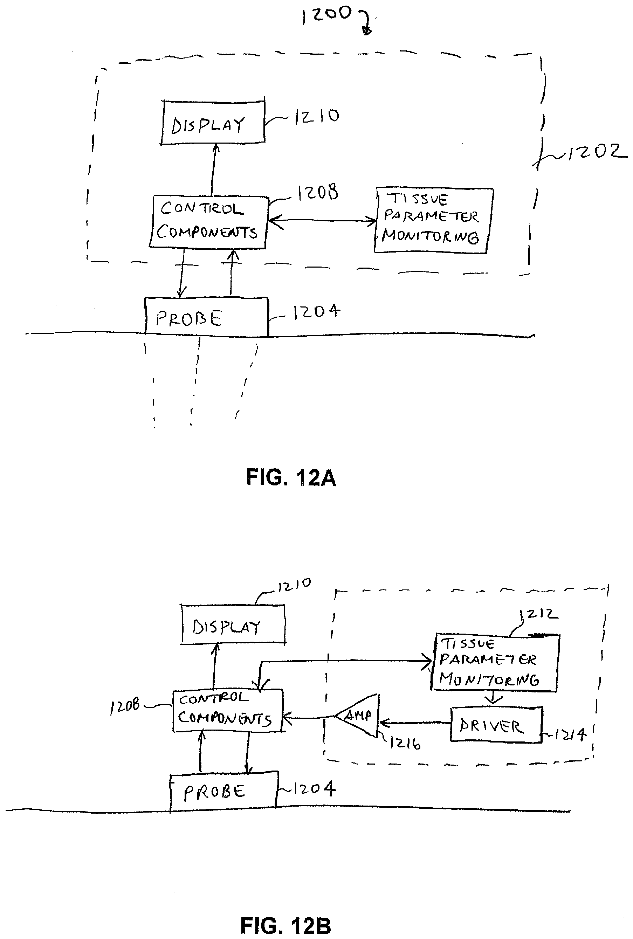

Such spatial and/or temporal control can also be enhanced through open-loop and/or closed loop feedback systems. For example, with reference to Fig. 12A, treatment system 1200 can comprise an open-loop feedback structure having a control system 1102 configured with a probe 1204 to treat a region-of-interest 1206. Control system 1202 can comprise control components 1208, such as the various control components within control system 300, a display 1210, and tissue parameter monitoring components 1212, or any other sensing or monitoring components. Display 1210 can comprise any display configured for illustrating images, such as of the treatment region, and/or any spatial or temporal parameter. In such an open-loop system, a system user can suitably monitor the imaging and or other spatial or temporal parameters and then adjust or modify same to accomplish a particular treatment objective. Instead of, or in combination with open-loop feedback configurations, with reference to an exemplary embodiment illustrated in Fig. 12B, an exemplary treatment system 1200 can comprise a closed-loop feedback system, wherein images and/or spatial/temporal parameters can be suitably monitored within monitoring component 1212 to generate signals, e.g., within a driver 1214 and amplifier 1216, or any other controllable aspect of treatment system 1100, to provide to control components 1208. As a result, a closed-loop control of the output and operation of probe 1204 can be realized.
During operation of an exemplary treatment system, a lesion configuration of a selected size, shape, orientation is determined. Based on that lesion configuration, one or more spatial parameters are selected, along with suitable temporal parameters, the combination of which yields the desired conformal lesion. Operation of the transducer can then be initiated to provide the conformal lesion or lesions. Open and/or closed-loop feedback systems can also be implemented to monitor the spatial and/or temporal characteristics, and/or other tissue parameter monitoring, to further control the conformal lesions.

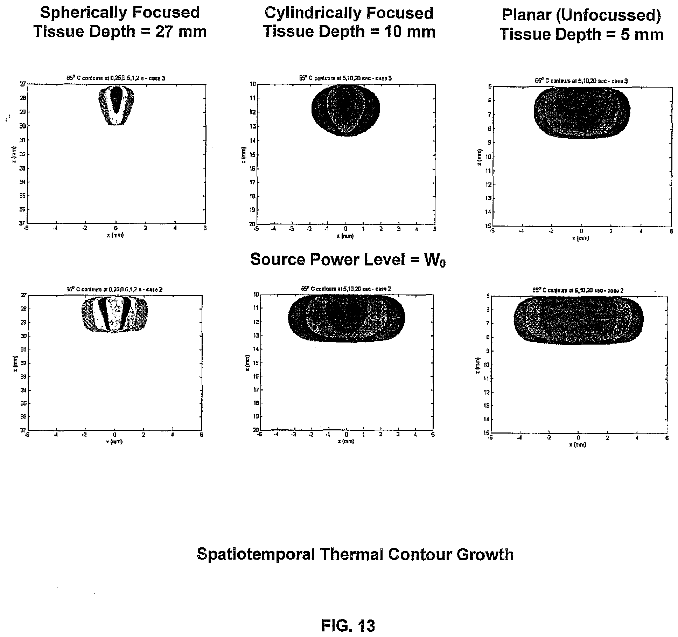

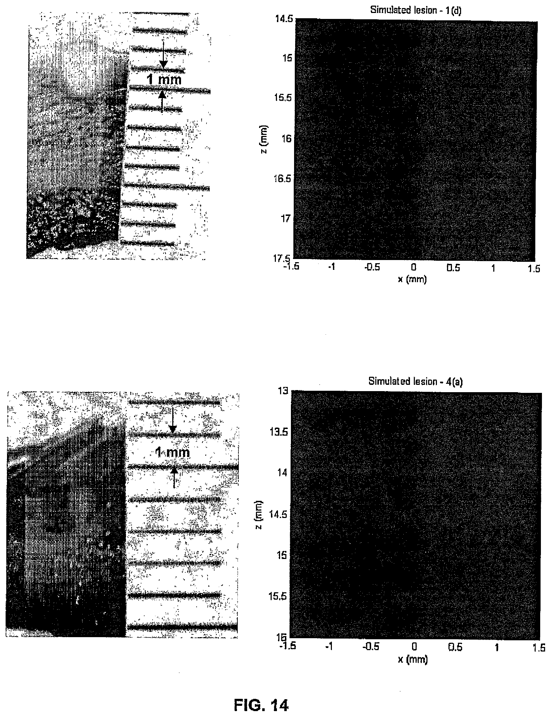

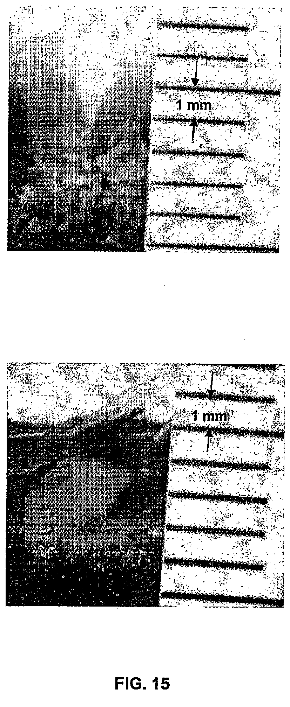

With reference to FIG. 13, a collection of simulation results, illustrating thermal lesion growth over time are illustrated. Such lesion growth was generated with a spherically focused, cylindrically focused, and planar (unfocused) source at a nominal source acoustic power level, W₀ and twice that level, 2 W₀, but any configurations of transducer can be utilized as disclosed herein. The thermal contours indicate where the tissue reached 65 °C for different times. The contour for the cylindrically focused source is along the short axis, or so-called elevation plane. The figure highlights the different shapes of lesions possible with different power levels and source geometries. In addition, with reference to FIG. 14, a pair of lesioning and simulation results is illustrated, showing chemically stained porcine tissue photomicrographs adjacent to their simulation results. In addition, with reference to FIG. 15, another pair of lesioning results is illustrated, showing chemically stained porcine tissue photomicrographs, highlighting a tadpole shaped lesion and a wedge shaped lesion.

In summary, adjustment of the acoustic field spatial distribution via transducer type and distribution, such as size, element configuration, electronic or mechanical lenses, acoustic coupling and/or cooling, combined with adjustment of the temporal acoustic field, such as through control of transmit power level and timing, transmit frequency and/or drive waveform can facilitate the achieving of controlled thermal lesions of variable size, shape, and depths. Moreover, the restorative biological responses of the human body can further cause the desired effects to the superficial human tissue.

## Claims

1. A therapeutic treatment system for controlled thermal injury in human superficial tissue within a region-of-interest (200), said treatment system comprising:
a control system (102) configured for control of said treatment system;
a probe system (104) configured for generating a conformal lesion within the region-of-interest, said control system and said probe system being configured for spatial and temporal control to generate the conformal lesion, the probe system comprising:
a single ultrasound therapy element composed of one individual single-element transducer, configured for single focus and for treating said region-of-interest, the single ultrasound therapy element configured for generating the conformal lesion within the region-of-interest (200) at the depth of superficial human tissue between zero and 30 mm from a skin surface; and
a motion mechanism in the probe system for controllably creating a plurality of conformal lesions,
a transducer probe housing or arrangement for coupling to a tissue interface, wherein the probe system (104) is configured within the transducer probe housing or arrangement; and
the control system (102) being in communication with and controlling said probe system (104) and being configured to controllably produce the plurality of conformal lesions separated throughout the region-of-interest (200).

2. The therapeutic treatment system according to claim 1, wherein said spatial control comprises operational control of one or more spatial parameters comprising transducer configuration, distance, placement, orientation, and movement.

3. The therapeutic treatment system according to any of the previous claims, wherein said temporal control comprises operational control of one or more temporal parameters comprising drive amplitude levels, frequency/waveforms, and timing sequences.

4. The therapeutic treatment system according to any of the previous claims, wherein said spatial and temporal control is facilitated through an open-loop feedback control loop.

5. The therapeutic treatment system according to any of the previous claims, wherein said spatial and temporal control is facilitated through a closed-loop feedback control loop.

6. The therapeutic treatment system according to any of the previous claims, wherein said control system comprises: power source components configured to provide energy to said control system and said probe system; sensing and monitoring components configured for monitoring said spatial and temporal parameters; cooling and coupling controls configured to facilitate temperature control at superficial human tissue interface and deeper into tissue; and processing and control logic components for overall control of said therapeutic treatment system.

7. The therapeutic treatment system according to any of the previous claims, wherein said probe system comprises: a control interface for interfacing with said control system; coupling components for acoustically coupling said transducer to the region of interest; monitoring and sensing components for facilitating control by said control system.

8. The therapeutic treatment system of claim 7, wherein said probe system comprises at least one of sensors, motion sensors, switches, actuators, indicators, encoders, probe identification, memory devices, aperture selection and multiplexers, electric matching networks, and switching, interconnect, connectors, handle, and disposable members.

9. The therapeutic treatment system according to claim 6, wherein said control system comprises at least one of treatment planning, display, switches, microcontroller, microprocessor, computer, control firmware, user and control software.

10. The therapeutic treatment system of claim 6, whereby said control system comprises one or more of DC power supplies, current sensors, power detection and measurement, waveform synthesizer, oscillator, digital programmable logic, amplifier/driver, beam former, harmonic filter, matching networks, cooling/acoustic coupling/thermal control/acoustic focusing control hardware, motion mechanism drivers and control, motion sensors, monitoring and closed loop control, interfacing and control, external input/output hardware and external interfaces.

11. The therapeutic treatment system according to claim 6, wherein said probe system comprises transducer acoustic coupling, cooling, and/or liquid-filled acoustic lensing mechanisms configured to regulate temperature at an interface between said transducer and the interest, and into region-of-interest.

12. The therapeutic treatment system according to claim 2 and any of the previous claims, wherein said transducer configuration comprises a convex-shaped lens, in the form of at least one of a liquid-filled, gel-filled, solid gel lens, rubber or composite lens or any material with velocity less than that of superficial tissue and/or acoustic coupling medium and with adequate power handling capacity.

13. The therapeutic treatment system according to claim 2 and any of the previous claims, wherein said transducer configuration comprises a combined imaging/therapy transducer within a single transduction element, wherein said imaging transducer is electrically isolated from said therapy transducer.

14. The therapeutic treatment system according to any of the previous claims, wherein said control system comprises an imaging system configured for facilitating at least one of one- dimensional, two-dimensional and three-dimensional imaging or treatment.

## Patentansprüche

1. Ein therapeutisches Behandlungssystem für kontrollierte thermische Verletzung in humanem oberflächlichem Gewebe innerhalb einer *Region of Interest* (200), wobei das Behandlungssystem aufweist:
ein Steuerungssystem (102) konfiguriert zur Steuerung dieses Behandlungssystems;
ein Sondensystem (104) konfiguriert zur Generierung einer konformen Läsion innerhalb der *Region of Interest,* wobei dieses Steuerungssystem und dieses Sondensystem für eine räumliche und zeitliche Steuerung konfiguriert sind, um die konforme Läsion zu generieren, wobei das Sondensystem aufweist:
ein einzelnes Ultraschalltherapieelement aufgebaut aus einem individuellen Einzelelement-Transducer, der für einen Einzelfokus konfiguriert ist und dazu konfiguriert ist, diese *Region of Interest* zu behandeln, wobei das einzelne Ultraschalltherapieelement dazu konfiguriert ist, die konforme Läsion innerhalb dieser *Region of Interest* (200) in der Tiefe von oberflächlichem humanem Gewebe zwischen null und 30 mm entfernt von einer Hautoberfläche zu erzeugen; und
einen Bewegungsmechanismus in dem Sondensystem, um eine Vielzahl von konformen Läsionen kontrollierbar zu erzeugen,
einem Transducersondengehäuse oder eine Anordnung zur Kopplung an eine Gewebegrenzfläche, wobei das Sondensystem (104) innerhalb des Transducersondengehäuses oder innerhalb der Anordnung konfiguriert ist; und
wobei das Steuerungssystem (102) in Verbindung mit diesem Sondensystem (104) steht und dieses steuert und dazu konfiguriert ist, die Vielzahl von konformen Läsionen (200) kontrolliert zu erzeugen, die in der *Region of Interest* voneinander beabstandet sind.

2. Das therapeutisches Behandlungssystem nach Anspruch 1, wobei diese räumliche Steuerung eine operative Steuerung von einem oder mehreren räumlichen Parametern bestehend aus Transducerkonfiguration, Distanz, Platzierung, Orientierung und Bewegung enthält.

3. Das therapeutische Behandlungssystem nach einem der vorangehenden Ansprüche, wobei diese zeitliche Steuerung eine operative Steuerung von einem oder mehreren zeitlichen Parametern aufweist, die den Antriebsamplitudenlevel, die Frequenz/Wellenformen und zeitliche Abläufen umfassen.

4. Das therapeutische Behandlungssystem nach einem der vorangehenden Ansprüche, wobei diese räumliche und zeitliche Steuerung durch einen Regelkreis mit offener Rückkopplung ermöglicht ist.

5. Das therapeutische Behandlungssystem nach einem der vorangehenden Ansprüche, wobei diese räumliche und zeitliche Steuerung durch einen Regelkreis mit geschlossener Rückkopplung ermöglicht ist.

6. Das therapeutische Behandlungssystem nach einem der vorangehenden Ansprüche, wobei das Steuerungssystem aufweist: Energiequellenkomponenten konfiguriert um Energie an dieses Steuerungssystem und dieses Sondensystem zu liefern; Sensorik- und Überwachungskomponenten konfiguriert zur Überwachung dieser räumlichen und zeitlichen Parameter; Kühl- und Kopplungssteuerungen konfiguriert um Temperaturkontrolle an oberflächlichen humanen Gewebegrenzflächen und tiefer im Gewebe zu ermöglichen und Verarbeitungs- und Steuerlogikkomponenten zur Gesamtsteuerung dieses therapeutischen Behandlungssystems.

7. Das therapeutische Behandlungssystem nach einem der vorangehenden Ansprüche, wobei dieses Sondensystem aufweist: eine Steuerungsschnittstelle zur Verbindung mit diesem Steuerungssystem; Kopplungsteile zur akustischen Kopplung dieses Transducers zur *Region of Interest*; Überwachungs- und Sensorikkomponenten, um die Steuerung durch dieses Steuerungssystems zu ermöglichen.

8. Das therapeutische Behandlungssystem nach Anspruch 7, wobei dieses Sondensystem mindestens eine der folgenden Komponenten aufweist: Sensoren, Bewegungssensoren, Schalter, Stellantriebe, Anzeigen, Encoder, Sondenidentifikation, Datenspeichergeräte, Blendenselektion und Multiplexer, elektrisches Anpassungsnetz, und Umschaltung, Verbindungen, Steckverbinder, einen Griff, und Einwegteile.

9. Das therapeutische Behandlungssystem nach Anspruch 6, wobei dieses Steuerungssystem mindestens eine der folgenden Komponenten enthält: eine/n Behandlungsplanung, Display, Schalter, Mikrokontroller, Mikroprozessoren, Computer, Steuerungs-Firmware, Anwender- und Steuerungssoftware.

10. Das therapeutische Behandlungssystem nach Anspruch 6, wobei dieses Steuerungssystem eine oder mehrere der folgenden Komponenten aufweist: eine/n oder mehrere DC-Netzteile, Stromfühler, Stromdetektion und Messung, Wellenform-Synthesizer, Oszillator, digitale programmierbare Logik, Verstärker/Treiber, Strahlformer, Oberschwingungsfilter, Anpassungsnetzwerke, Kühlung/Akustik koppelnd/thermisch kontrollierende/akustische fokussierende Steuerungshardware, Bewegungsmechanismus Treiber und Steuerung, Bewegungssensoren, Überwachung und geschlossene Regelkreise, Schnittstellen und Steuerung, externe Input/Output-Hardware und externe Schnittstellen.

11. Das therapeutische Behandlungssystem nach Anspruch 6, wobei dieses Sondensystem eine Transducer-Akustik-Kopplung, Kühlung, und/oder flüssigkeitsgefüllte akustische Linsenmechanismen enthält, konfiguriert um die Temperatur an einer Grenzfläche zwischen diesem Transducer und dem Ziel und in der *Region of Interest* zu regulieren.

12. Das therapeutische Behandlungssystem nach Anspruch 2 und einem der vorangehenden Ansprüche, wobei die genannte Transducerkonfiguration eine konvex geformte Linse enthält, in der Form von mindestens einer flüssigkeitsgefüllten, gelgefüllten, festen Gellinse, Elastik- oder Verbundmateriallinse oder jedem Material mit einer geringeren Geschwindigkeit als die des oberflächlichem Gewebes, und/oder ein akustisches Kopplungsmittel, und eine angemessene Stromaufnahme-Belastbarkeit.

13. Das therapeutische Behandlungssystem nach Anspruch 2 und einem der vorangehenden Ansprüche, wobei die genannte Transducerkonfiguration einen kombinierten Bildgebungs-/Therapie-Transducer in einem einzelnen Transduktionselement enthält, wobei dieser Bildgebungstransducer elektrisch isoliert ist von diesem Therapietransducer.

14. Das therapeutische Behandlungssystem nach einem der vorangehenden Ansprüche, wobei dieses Steuerungssystem ein Bildgebungssystem enthält, welches konfiguriert ist um mindestens eine ein-dimensionale, zwei-dimensionale und drei-dimensionale Bildgebung oder Behandlung zu ermöglichen.

## Revendications

1. Système de traitement thérapeutique pour la lésion thermique contrôlée de tissus superficiels humains dans une région d'intérêt (200), ledit système de traitement comprenant :
un système de contrôle (102) configuré pour le contrôle dudit système de traitement,
un système de sonde (104) configuré pour générer une lésion conformée dans la région d'intérêt, ledit système de contrôle et ledit système de sonde étant configurés pour un contrôle spatial et temporel afin de générer la lésion conformée, le système de sonde comprenant :
un élément de traitement par ultrasons unique composé d'un transducteur à élément unique individuel, configuré pour la focalisation unique et pour le traitement de ladite région d'intérêt, l'élément de traitement par ultrasons unique étant configuré pour générer la lésion conformée à l'intérieur de la région d'intérêt (200) à la profondeur du tissu humain superficiel comprise entre zéro et 30 mm à partir d'une surface cutanée, et
un mécanisme de mouvement dans le système de sonde pour créer de manière contrôlable une pluralité de lésions conformées,
un boîtier de sonde de transducteur ou un arrangement destiné à être couplé à une interface tissulaire, dans lequel le système de sonde (104) est configuré à l'intérieur du boîtier de sonde de transducteur ou l'arrangement, et
le système de contrôle (102) étant en communication avec ledit système de sonde (104) et le contrôlant et étant configurée pour produire de manière contrôlable la pluralité de lésions conformées séparées dans toute la région d'intérêt (200) .

2. Système de traitement thérapeutique selon la revendication 1, dans lequel ledit contrôle spatial comprend le contrôle opérationnel d'un ou plusieurs paramètres spatiaux comprenant la configuration, la distance, le placement, l'orientation et le mouvement du transducteur.

3. Système de traitement thérapeutique selon l'une quelconque des revendications précédentes, dans lequel ledit contrôle temporel comprend le contrôle opérationnel d'un ou plusieurs paramètres temporels comprenant les niveaux d'amplitude de pilotage, la fréquence, les formes d'onde et les séquences de temporisation.

4. Système de traitement thérapeutique selon l'une quelconque des revendications précédentes, dans lequel ledit contrôle spatial et temporel est facilité par une boucle de contrôle à rétroaction en boucle ouverte.

5. Système de traitement thérapeutique selon l'une quelconque des revendications précédentes, dans lequel ledit contrôle spatial et temporel est facilité par une boucle de contrôle à rétroaction en boucle fermée.

6. Système de traitement thérapeutique selon l'une quelconque des revendications précédentes, dans lequel ledit système de contrôle comprend :
des composants de source d'alimentation configurés pour fournir de l'énergie audit système de contrôle et audit système de sonde ;
des composants de détection et de surveillance configurés pour surveiller lesdits paramètres spatiaux et temporels ;
des contrôles de refroidissement et de couplage configurés pour faciliter la régulation de la température au niveau de l'interface tissulaire humaine superficielle plus profondément dans le tissu ; et
des composants logiques de traitement et de contrôle pour le contrôle global dudit système de traitement thérapeutique.

7. Système de traitement thérapeutique selon l'une quelconque des revendications précédentes, dans lequel ledit système de sonde comprend : une interface de contrôle destinée à être en interface avec ledit système de contrôle ; des composants de couplage pour le couplage acoustique dudit transducteur à la région d'intérêt ; et des composants de surveillance et de détection destinés à faciliter le contrôle par ledit système de contrôle.

8. Système de traitement thérapeutique selon la revendication 7, dans lequel ledit système de sonde comprend au moins un parmi des capteurs, des capteurs de mouvement, des commutateurs, des actionneurs, des indicateurs, des encodeurs, un identificateur de sonde, des dispositifs de mémoire, un appareil de sélection d'ouverture et des multiplexeurs, des réseaux d'adaptation électrique, des connecteurs de basculement et d'interconnexion, des éléments jetables de maintien.

9. Système de traitement thérapeutique selon la revendication 6, dans lequel ledit système de contrôle comprend au moins parmi un plan de traitement, un écran, des commutateurs, un microcontrôleur, un micro-processeur, un ordinateur, un micro-logiciel de contrôle, et un logiciel de contrôle et d'utilisateur.

10. Système de traitement thérapeutique selon la revendication 6, dans lequel ledit système de contrôle comprend au moins un ou plusieurs éléments parmi des alimentations en courant continu, des capteurs de courant, un appareil de détection de puissance et de mesure, un synthétiseur de forme d'onde, un oscillateur, une logique programmable numérique, un amplificateur/pilote, un formateur de faisceau, un filtre d'harmonique, des réseaux d'adaptation, un matériel de contrôle de focalisation acoustique/régulation thermique/couplage acoustique /refroidissement, des pilotes et un contrôle de mécanisme de mouvement, des capteurs de mouvement, un appareil de suivi et de contrôle de boucle fermée, un matériel d'interface et de commande d'entrées/sorties externe, des interfaces externes.

11. Système de traitement thérapeutique selon la revendication 6, dans lequel ledit système de sonde comprend un appareil de couplage acoustique de transducteur, un appareil de refroidissement, et/ou un mécanisme lenticulaire acoustique rempli de liquide configuré pour réguler la température entre ledit transducteur et l'intérêt, et dans la région d'intérêt.

12. Système de traitement thérapeutique selon la revendication 2 et l'une quelconque des revendications précédentes, dans lequel ladite configuration de transducteur comprend une lentille de forme convexe, se présentant sous la forme d'au moins un élément parmi une lentille en gel solide, remplie de gel, remplie de liquide, une lentille en caoutchouc ou composite ou un matériau quelconque présentant une vitesse inférieure à celle du tissu superficiel et/ou d'un support de couplage acoustique et présentant une capacité productive adéquate.

13. Système de traitement thérapeutique selon la revendication 2 et l'une quelconque des revendications précédentes, dans lequel ladite configuration de transducteur comprend un transducteur d'imagerie/de traitement combiné à l'intérieur d'un élément de transduction unique, dans lequel ledit transducteur d'imagerie est isolé électriquement dudit transducteur de traitement.

14. Système de traitement thérapeutique selon l'une quelconque des revendications précédentes, dans lequel ledit système de contrôle comprend un système d'imagerie configuré pour faciliter au moins un parmi une imagerie ou un traitement unidimensionnel(le), bidimensionnel(le) et tridimensionnel(le).
